**Europäisches Patentamt**

**European Patent Office**

**Office européen des brevets**

(11) Publication number: **0 394 041**
**A2**

## EUROPEAN PATENT APPLICATION

(21) Application number: **90304217.4**

(22) Date of filing: **19.04.90**

(51) Int. Cl.5: **G01N 33/48, G01N 21/49, G01N 11/04**

(30) Priority: **20.04.89 US 341079**

(43) Date of publication of application:
**24.10.90 Bulletin 90/43**

(84) Designated Contracting States:
**AT BE CH DE DK ES FR GB GR IT LI LU NL SE**

(71) Applicant: **Biotrack, Inc.**
**1058 Huff Avenue**
**Mountain View California 94043(US)**

(72) Inventor: **Lingane, Paul James**
**352 Calcatera Place**
**Palo Alto, California 94306(US)**
Inventor: **Carey, Brian Robert**
**1065 Paintbrush Drive**
**Sunnyvale, California 94086(US)**

(74) Representative: **Harrison, David Christopher**
**et al**
**MEWBURN ELLIS 2 Cursitor Street**
**London EC4A 1BQ(GB)**

(54) Capillary flow device and reading instrument.

(57) An instrument for performing assays is taught including one or more of the following features: an APTT test is performed utilizing a bandpass filter centered at approximately 45Hz in order to provide the best analysis of scattered light for properly detecting the endpoint of the APTT assay; a floating theshold voltage is used for comparison with the output signal, thereby avoiding problems which cause false endpoint detection; periodic determinations are made to ensure that an adequate supply of sample is present in the sample well, thereby avoiding false indications of an adequate supply of sample due to sample drying; pulses of light are used rather than modulated light, thereby obviating the need for a modulator while providing scattered light from a sample which can be easily distinguished from ambient light; unique codes contained on a quality check device are utilized for customization, for example, to establish the language in which the prompts will be presented to the user, or the units of measurement which will be displayed; and a thermal code is used to control the temperature of the cartridge containing the sample and one or more reagents within very close tolerances.

FIG. 3

## CAPILLARY FLOW DEVICE AND READING INSTRUMENT

This invention is related to testing devices having internal chambers in which fluids move, to methods of using such devices, and to apparatuses used to monitor reactions in such devices.

In the development of the diagnostics field, there has been explosive growth in the number of substances to be determined. For the most part, the medical field has looked to clinical laboratories for these determinations. The clinical laboratories have been dependent upon expensive, sophisticated equipment and highly trained technical help to fulfill the manifold needs of the medical community. However, in a highly automated clinical laboratory, there is substantial need to perform one or a few assays on a stat basis with minimum equipment.

There is also an expanding need for having analytical capabilities in "distributed sites" such as doctor's offices, emergency rooms, and in the home. The key is a need for test results which can be obtained objectively, quickly, preferably while the patient is still present, and in a convenient, fail-safe form so that skill is not a requirement for running a test and obtaining the test results. There is a continuing need to monitor the level of drug administered to people with chronic illnesses, such as diabetics, asthmatics, epileptics, and cardiac patients, as it appears in a physiological fluid, such as blood. Tests of interest include prothrombin time, potassium ion, and cholesterol. Determining red-blood-cell count is also a common test. In the case of diabetic patients, it is necessary to determine sugar level in urine or blood.

Numerous approaches have been developed toward this end, depending to varying degrees on instrumental or visual observation of the result. Typical of these are the so called "dip-stick" methods. These methods typically employ a plastic strip with a reagent-containing matrix layered thereon. Sample is applied to the strip and the presence or absence of an analyte is indicated by a color-forming reaction. While such devices have proven useful for the qualitative determination of the presence of analytes in urine and can even be used for rough quantitative analysis, they are not particularly useful with whole blood because of the interferring effects of red blood cells, nor are they useful for making fine quantitative distinctions. Accordingly, there remains a need for the development of methods and devices capable of analyzing whole blood and other complex samples rapidly with a minimum of user manipulations.

U.S. Patent No. 4,756,884, issued to Hillman et. al. and assigned to Biotrack Corporation, the assignee of this application, teaches various embodiments of fabricating devices including a defined chamber or channel, the resulting devices, and techniques for use of such device in analyzing a fluid such as blood. By "analyte" is meant either a chemical component of the sample or a physical or chemical (biochemical) property of the sample that is being detected or measured. Examples of chemical analytes include electrolytes such as sodium and potassium; drugs, such as dilantin and heparin; and biochemical components, such as cholesterol and enzymes. Examples of properties that can be analyzed include blood clotting and sample viscosity. A device within the scope of this patent suitable for use in measuring prothrombin time (PT) has been commercially produced under the name Protime. However, this commercial device was not optimized for measuring activated partial thromboplastin time (APTT), a measure of a different aspect of blood clotting. The monitor into which the Protime cartridge is inserted for analysis utilizes a light source, such as a laser, for providing light to the blood sample, and read transmitted light in order to determine when blood flow has ceased, i.e, when the endpoint of the prothrombin reaction has been reached and blood ceases to flow through the capillary due to clot formation. The Protime monitor uses a fixed threshold voltage for comparing a voltage related to the the optical signal, in order to detect the endpoint of the prothrombin assay. The commercial prothrombin time cartridge has now been modified to allow use in an APTT assay. This new cartridge is described in copending application serial no.    , filed    , and entitled "Capillary Flow Device for Measuring Activated Partial Thromboplastin Time", and bearing attorney docket no. BIOT-18. However, it has been determined by the inventors that while the fixed threshold voltage used in the prothrombin time analysis allows proper endpoint detection, a fixed threshold voltage results in somewhat imprecise endpoint detection when blood flow through the current commercial design of the APTT capillary device system is temporarily slowed, for example, by something less than complete clotting, during the significantly longer analysis time required of the APTT assay. The use of a fixed threshold voltage has also been discovered by the inventors to be undesirable due to the fact that there is often times a wide variation in signal level from sample to sample, on the order of 3 or more, due to varying hematocrit levels (i.e. the concentration of red blood cells in a blood sample) and lipemic samples (i.e. the concentration of fats in the blood samples).

Another issue encountered with the current APTT cartridge design noted by the inventors is that blood flow may have ceased due to insufficient fluid blood in the application well, but that the dried blood residue contained in the application well provides scattered light which falsely indicates that there is sufficient blood in the well to continue the test. In this case the monitor would not detect that there was insufficient blood in the application well to continue the test. For relatively long tests such as an APTT assay, if a detection for the presence of blood in the sample well is performed when it is believed the endpoint has been reached, dried blood residue will provide a false signal erroneously indicating that the sample well still contains blood, and that the test has been successfully completed.

The PT monitor utilizes a relatively massive platen which is heated to a constant temperature in order to cause the cartridge to be held at the appropriate test temperature. While this temperature control works well for prothrombin assays, it provides inadequate control for more temperature sensitive tests, such as APTT assays.

It is often desirable or necessary to customize the performance or output of an instrument to a specific installation. For example, for an instrument that will be sold into several countries with different languages, it is necessary to specify the language for any user prompts, error messages, and results output. This is particularly true for a medical instrument where a prompt or output that cannot be readily understood can lead to an improper medical action. Different countries also employ varying measurement units in reporting medical test results. Unless the result format is customized, the result might be in unaccustomed units that could lead to confusion or even incorrect medical action.

Instrument customization can be performed by the manufacturer; however, this requires that the manufacturer maintain several instruments in inventory that are identical except for the customization. This is costly and inefficient for the manufacturer. This approach also precludes the user from later customization, should that be required.

Another method of customization that has been employed in the prior art is with mechanical switches on the instrument. If these switches are set by a service technician, a service call is required for customization. If these switches are set by the customer, it requires that the customer understand the purpose and interactions of the various switches. A non-technical customer may find the setting of switches difficult or even impossible to perform. A lack of user understanding can lead to errors in the settings and, consequently, erroneous output.

An alternative method of customization is through an interactive menu where the user or a factory technician goes through a setup menu making selections among various options. Although typically easier than setting switches, this approach is still imperfect. It requires a technician visit with customer interaction for the setup, or else the customer is required to perform other complex selections without a full understanding of the ramifications. If the customer makes mistakes in this setup, unpredictable results may occur. The menu setup approach also has the disadvantage that if power is lost the setup can be lost and will have to be repeated.

It is also commonly necessary to check the performance of or to calibrate an instrument, particu larly an instrument employed in medical practice. This check is often through the use of an external device that is inserted into the instrument. For example, U.S. Patent 4,756,884 discloses the use of an electronic control device that is inserted into the device to confirm the performance of the system. It is recommended that this electronic control be run frequently. Often, this electronic control device also contains a code, either mechanical, optical (such as a bar code) or magnetic which, for example, indicates certain calibration or instrument set up information. An instrument which reads this code from the electronic control device is also capable of reading a code from the device containing analyte being read by the instrument, indicating lot-to-lot variations of cartridges, including variations in optical characteristics and/or variations in reagents contained within the cartridge and to identify the test type.

In accordance with one embodiment of this invention, an APTT test is performed utilizing a bandpass filter centered at approximately 45 Hz in order to provide the best analysis of scattered light for properly detecting the endpoint of the APTT assay. In another embodiment of this invention, a floating threshold voltage is used for comparison with the output signal, thereby avoiding problems which cause false endpoint detection.

In one embodiment of this invention, periodic determinations are made to ensure that an adequate supply of sample is present in the sample well, thereby avoiding false indications of an adequate supply of sample due to sample drying.

In one embodiment of this invention, pulses of light are used rather than modulated light, thereby obviating the need for a modulator while providing scattered light from a sample which can be easily distinguished from ambient light. As yet another feature of this invention, the device is capable of being customized utilizing unique codes contained on a quality check device, for example, to establish the language in which the prompts will be presented to the user, or the units of measurment which will be displayed. In another embodiment of this invention, a thermal model is used to control the temperature of the cartridge containing the sample and one or more reagents within very close tolerances.

In the drawings:

Figure 1 is a diagram depicting a relationship between signal level and threshold voltage during an assay;

Figure 2 is a graph depicting the signal level from the sample well during the performance of an assay;

Figure 3 is a flow chart depicting one embodiment of an algorithm used to adjust the threshold voltage, as depicted in Figure 1; and

Figure 4 is a flow chart depicting one embodiment of a thermal model used to maintain cartridge temperature at a desired level.

As described in U.S. Patent No. 4,756,884, by illuminating the blood sample, the measurement of light, e.g. scatter, can be used to measure a change in the rate of flow of blood, as in a capillary track. Unclotted blood, while flowing, provides an electrical signal which appears to be broadband noise, while with decreased flow rate, as blood begins to clot, the spectrum of this noise collapses to lower and lower frequencies. In performing the prothrombin time assay, a high-pass filter with cutoff at approximately 100 Hz is used to detect when the spectrum has sufficiently collapsed to indicate prothrombin endpoint.

In accordance with the teachings of this invention, activated partial thromboplastin time (APTT) is determined utilizing a cartridge having a longer capillary length (in one embodiment approximately three times as long) as the path length used in a cartride for determining prothrombin time. In one embodiment of this invention, capillary path length in the APTT cartridge is within the range of approximately 18 to 31 centimeters. It has been empirically determined by the inventors that utilizing a bandpass filter centered at approximately 45 Hz provides the best analysis of the scattered light for detecting the endpoint of the APTT assay. In one embodiment, this 45 Hz bandpass filter has a 3 dB bandwidth of approximately 35 Hz. As is appreciated by those of ordinary skill in the art, the 100 Hz highpass filter and the 45 Hz bandpass filter can be achieved using either hardware elements, or software control, or a combination of both.

In accordance with the teachings of the prior art, a fixed threshold voltage is used for comparison with the output signal from the high-pass filter used in the prothrombin time assay or the bandpass filter used for the APTT assay. In accordance with the teachings of this invention, a floating threshold voltage (typically approximately 0.5 volts very shortly after the assay is begun, although this value will vary by perhaps a factor of 2 or 3, depending on the signal level from the sample) is utilized in order to detect not merely the crossing of a fixed threshold voltage, but rather a pronounced and sustained crossing of a variable threshold voltage.

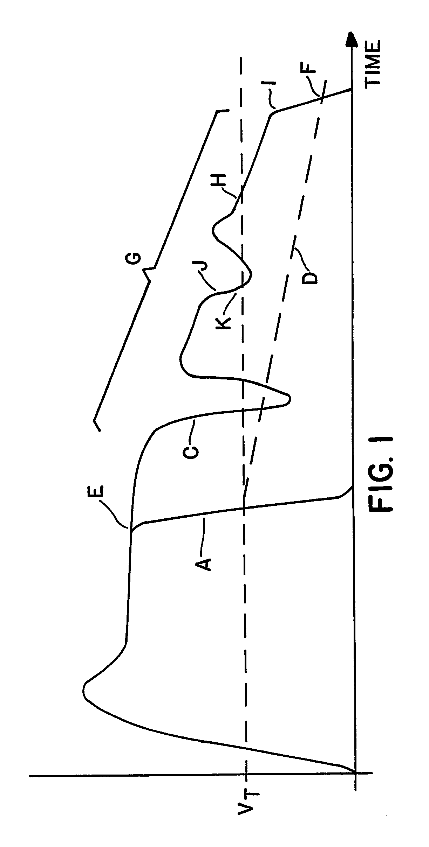

As shown in the graph of Figure 1, an ideal response curve A is shown, with signal fairly constant until knee E is reached at the endpoint. Utilizing a fixed threshold voltage $V_t$, E is easily detected as curve A decreases below threshold voltage $V_t$. However, with assays that require a longer period of time, such as the APTT assay, the signal gradually tapers off (as shown by curve G) as the flow rate slows down with increases in the capillary path length in the cartridge. Utilizing a fixed threshold voltage may result in erroneously calling the endpoint at point H rather than at the correct point F. Also, it can happen that the signal includes an undesired dip C or variation J caused by a temporary obstruction, for example, caused by partial clotting, which temporarily decreases capillary flow, following which capillary flow is increased. Utilizing a fixed threshold voltage $V_t$ causes an erroneous reading due to undesired dip C or variation J.

In accordance with the teachings of this invention, this problem is overcome by using a variable threshold voltage (curve D) which starts off at threshold voltage $V_t$, and is subsequently reduced in order to track the gradual decrease in signal level (curve G). In one of the embodiments of this invention, the signal at the start of an assay is relatively low, but quickly builds up to a maximum value, and then tapers off over time, as shown in the plot of Figure 1. In another embodiment of this invention, false triggering is avoided by waiting a predetermined period of time before comparing the signal with the threshold voltage. Another embodiment, the floating threshold voltage, is initialized to a rather low value, thereby assuring that the endpoint will not be falsely triggered at the beginning of the assay, due to the relatively low signal level. The threshold voltage is then made to track the input signal level, and thus rises rather quickly at the beginning of the assay, as shown in Fig. 1. Since floating threshold voltage curve D remains below signal curve G by a sufficient amount, undesired variation J, which dips below a fixed threshold $V_t$ at point K, does not trigger an endpoint indication. However, since the true endpoint, which occurs at knee I, causes a very significant decrease in signal curve G, the true endpoint is indicated at point F, where signal curve G falls below floating threshold voltage curve D.

In accordance with one embodiment of this invention, false indications of endpoint due to dip C are avoided by testing to determine whether the signal curve G has fallen below floating threshold voltage curve D for a minimum period of time, in one embodiment 10 seconds. Although one embodiment has been described which utilizes software to control the level of the floating threshold voltage, the floating threshold voltage can also be controlled in a similar manner utilizing hardware elements.

As previously described, endpoint detection is determined when capillary flow of blood being tested ceases, presumably due to clotting, as desired. In order to determine that a proper endpoint has been reached, i.e that fluid flow has ceased due to clotting, it must be determined that there remains adequate fluid blood in the sample reservoir, i.e. that fluid flow has not ceased due to depletion of blood in the sample reservoir. In order to determine if adequate blood remains in the sample reservoir, another optical source is utilized to provide light to the sample well, and a corresponding detector is used to measure the amount of light scattered from the sample well.

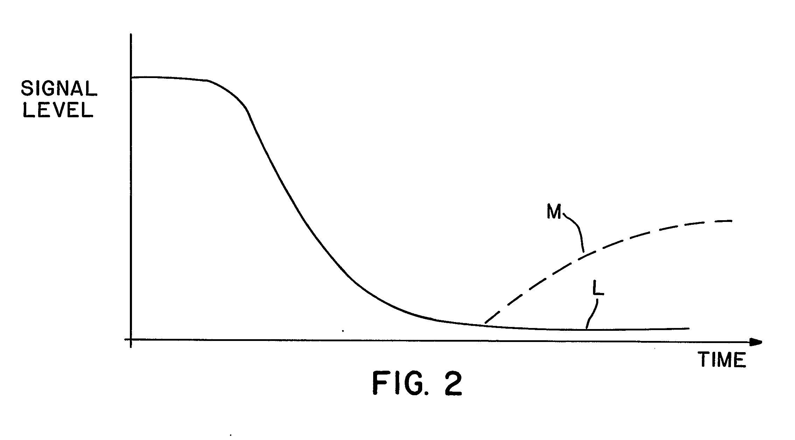

Figure 2 is a graph depicting the signal level from the sample well detector with respect to time, indicating a decrease in the amount of blood in the sample well. Curve L shows the ideal curve wherein the signal level decreases to a point near zero indicating that the sample well is empty, i.e that insufficient blood was present to allow capillary flow until capillary flow ceased due to clotting. In the prior art, when performing prothrombin assays, the assay was run until capillary flow ceased, and then a determination was made as to whether there was sufficient blood remaining in the sample well to indicate that flow ceased due to clotting, rather than due to lack of sample. No problems caused by sample drying were encountered, since the assay is complete in about 10 to 60 seconds.

However, for longer assays, such as APTT assays, there is a potential problem with the blood in the sample well drying, providing a false indication of blood remaining in the sample well at the time capillary flow ceases, thereby giving a false indication that the true endpoint has been reached. Curve M shows the undesired signal curve resulting from dried blood, i.e. over time it dries and provides increased signal which can be confused with blood remaining in the sample well.

In accordance with one embodiment this invention, the signal curve L is monitored periodically rather than simply interrogated when capillary flow ceases, in order to detect if the sample well fails to contain sufficient blood to complete the assay. In one embodiment of this invention, with an assay test requiring up to approximately 260 seconds, the presence of blood in the sample well is periodically detected by monitoring curve L at intervals of approximately 2 seconds. If, at any one of these interrogations of the sample well, it is determined that the sample well has run out of blood, the test is aborted. If desired, the user then performs a new test, performed hopefully with sufficient blood to reach a true endpoint due to clotting, rather than a lack of blood in the sample well.

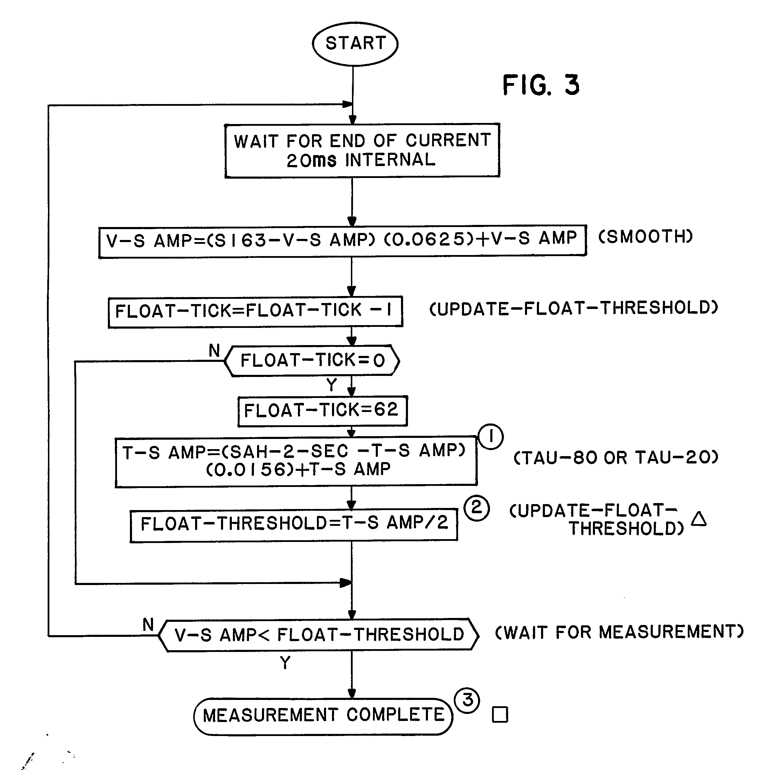

Figure 3 is a flow chart depicting the operation of one embodiment of this invention which is further described in the software code of Appendix 1. Notes pertaining to the flowchart of Figure 3 are as follows:

1. Multiplier changes from 0.0625 to 0.0156 after 40 seconds and variable SAH_2_SEC is copied from V_SAMP in interrupt service routine once every two seconds.

2. Firmware sets minimum value of FLOAT THRESHOLD to 100 millivolts

3. Firmware requires V_SAMPLE to be less than FLOAT THRESHOLD for 10 seconds before terminating measurement.

In prior art systems, modulated light was utilized in order to distinguish light emitted from the sample well (which lies outside of the instrument) from ambient light, which, of course, is uncontrollable in intensity and amplitude during the assay. As described in U.S. Patent No. 4,756,884 an oscillator circuit is utilized to modulate the emitted light such that detected light including this modulation can be electronically separated from ambient light. In accordance with one embodiment of the present invention, the need for such an oscillator is obviated and emitted light is not modulated, but rather pulsed, for example, with a pulse width of approximately 400 microseconds and a pulse repetition rate of approximately 1 pulse every 2 seconds. Since the pulse includes relatively fast rise and fall times (typically 7μsec) the pulse is rich in frequencies other than those contained in ambient light which typically includes frequencies of either 50 or 60 Hz and harmonics. In one embodiment of this invention, the receiving circuitry is tuned to detect received light of approximately 8 kilohertz, as described in the aforementioned U.S. Patent No. 4,756,884 even though the emitted light is not modulated at 8 kilohertz, but rather consists of a series of pulses. Alternatively, light which is amplitude, frequency, or phase modulated, is used.

In one embodiment of this invention, the quality check device is used to automatically customize the instrument. This approach has several advantages. The manufacturer has only to stock various check devices, which are typically much less expensive than the instrument. A technician visit is not required in order to set the options. The setup is transparent to the user; the customer does not have to read any complicated instructions nor sort through a set of menus. Even if the batteries fail the setup is re-established when the customer runs the check device, which, regardless of its use in customization, must be run often as a quality control measure.

In the prior art, individual light transmission means were used to couple light between eight emitter/detector pairs and eight bits of optical code on the cartridges to indicate lot-to-lot variances in cartridge reagent qualities and proper cartridge type. In accordance with the teachings of this invention, one embodiment includes 16 detectors for reading 16 bits of optical data on the cartridge, using a single light source and, for example, a prism or light pipe for coupling light from the source to the cartridge. The use of 16 bits allows error detection or correction codes to be used, such as a Hamming code, in order to prevent errors due to erroneous readings of the optical codes. Furthermore, in one embodiment, this same optical code is used on the electronic control device to set the customization features. In one embodiment, there are six electronic control devices coded to customize the language displayed by the instrument. The manufacturing cost of the electronic control device is less than one tenth that of the instrument, making it much more cost effective to inventory six different electronic control devices rather than to inventory six different instruments. In addition, if the customer requires prompts in two different languages, he has only to order two different electronic control devices in order to customize his instrument as needed.

In an alternative embodiment of this invention, the code on the cartridges themselves are used both to customize the instrument during a particular sample analysis as well as to indicate lot-to-lot variations among the cartridges.

In one embodiment, a test is made to determine if excessive ambient light is present, which might cause an error in reading the optical code. This is accomplished by measuring light with the emitter turned off, and determining the level of ambient light. If the level of ambient light is excessive, an error message is displayed.

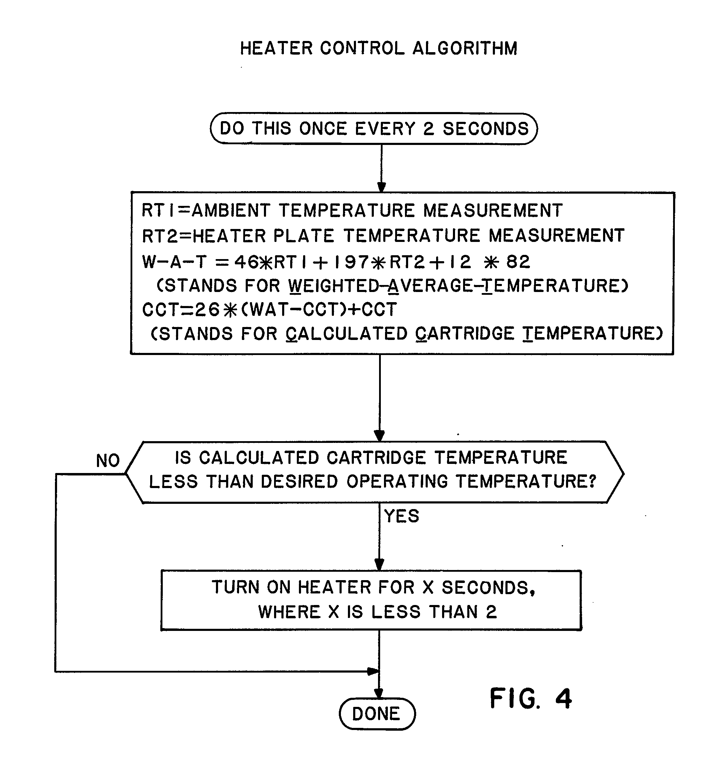

In one embodiment of this invention, rather than relying on heating a relatively massive platen in an attempt to maintain the cartridge temperature, as was done in the prior art, a thermal model is used to more accurately control the temperature of the cartridge and hence the sample and reagent contained therein. In accordance with one embodiment of this invention, a thermal model is used as depicted in the flowchart of Figure 4 and the associated source code shown in Appendix 2. As shown in Figure 4, RT1 and RT2 are numbers representing analog to digital converter counts, with each analog to digital converter count representing 20 millivolts. As depicted in Figure 4, W__A__T is the Weighted Average Temperature and C__C__T is the Calculated Cartridge Temperature. Utilizing this thermal model, the temperature within the cartridge is calculated based on the ambient temperature (preferably inside the measurement instrument, but ambient temperature outside the instrument can be used) and the heater plate channel temperature and the passage of time. This model is continuously updated, and the results of the model are used to decide if the heating element is to be turned on or off, to maintain the cartridge temperature to approximately $35.3 \pm 0.5°$ C. In one embodiment of this invention, thermistors are utilized to measure the temperatures used in the thermal model to calculate the temperature of the cartridge. Although one embodiment has been described which utilizes software to control temperature, temperature control can also be performed in a similar manner utilizing hardware elements.

In accordance with the teachings of one embodiment of this invention, the location of the sample detector is made quite close to the sample itself (typically approximately 0.4 cm), in direct contrast to the prior art which utilized optical transmission means such as mirrors in order to locate the detector at a distance (typically 10 cm) carefully selected such that the speckle size from the sample would be approximately equal to the size of the detector in order to maximize signal. In accordance with this embodiment of the present invention, the speckle size is much smaller than the size of the detector, providing significantly smaller signal levels (one embodiment signal levels are reduced by about a factor of about 200). Thus, in accordance with this embodiment, greater gain is provided by electronic circuitry and a lower noise light source is utilized.

All publications and patent applications cited in this specification are herein incorporated by reference as if each individual publication or patent application were specifically and individually indicated to be incorporated by reference.

Although the foregoing invention has been described in some detail by way of illustration and example for purposes of clarity of understanding, it will be readily apparent to those of ordinary skill in the art in light of the teachings of this invention that certain changes and modifications may be made thereto.

## Claims

1. A method for measuring an activity or determining an analyte in a fluid medium employing a device comprising at least one capillary unit acting as the motive force for moving the fluid medium in the device, at least one chamber unit, an inlet port, an outlet port distant from the inlet port, and a reagent contained within the device, said reagent being a member of a detection system, wherein said capillary acts as a metering pump and flow controller of the assay medium through said device to provide for a time controlled reaction with said reagent, said method comprising:

introducing a fluid sample though said inlet port into one of said units and allowing said fluid sample to transit from one unit to the next unit at a rate controlled by said capillary unit and react with said reagent;

determining a signal as a measure of said activity or the presence of concentration of said analyte in said sample;

passing said signal through a filter to provide an output signal;

detecting said output signal to provide a detected signal; and

comparing said detected signal with a threshold voltage to determine when said flow has stopped, said threshold voltage being adjusted to track said detected signal relatively slowly such that a rapid change in said detected signal will fall below said threshold voltage.

2. A method according to Claim 1, wherein said filter is a bandpass filter.

3. A method according to Claim 2, wherein said bandpass filter has a center frequency of approximately 45 Hz.

4. A method according to Claim 3, wherein said bandpass filter has a 3 dB bandwidth of approximately 35 Hz.

5. A method according to any one of the preceding claims wherein said analyte is an APTT assay.

·6. A method according to any one of the preceding claims wherein said detected signal must fall below said threshold voltage for a specified period of time to provide an indication that flow has stopped.

7. A method according to Claim 6, wherein said specific period of time is approximately 10 seconds.

8. A method for measuring an activity or determining an analyte in a fluid medium employing a device comprising at least one capillary unit acting as the motive force for moving the fluid medium in the device, at least one chamber unit, an inlet port, an outlet port distant from the inlet port, and a reagent contained within the device, said reagent being a member of a detection system, wherein said capillary acts as a metering pump and flow controller of the assay medium through said device to provide for a time controlled reaction with said reagent, said method comprising:

introducing a fluid sample though said inlet port into one of said units and allowing said fluid sample to transit from one unit to the next unit at a rate controlled by said capillary unit and react with said reagent resulting in a detectable signal produced by said detection system; and

determining said signal as a measure of said activity or the presence or concentration of said analyte in said sample,

wherein said detectable signal is a change in flow rate due to a stoppage of flow, and a periodic determination is made to insure that said stoppage of flow is not due to an inadequate supply of said fluid sample.

9. A method according to Claim 8 wherein said each of said periodic determinations comprises the steps of:

providing pulsed light to said fluid sample resulting in a detectable signal; and

determining a signal at a specified frequency as an indication of the state of said sample.

10. A method according to Claim 9, wherein said specified frequency is selected to reduce undesired signal components due to ambient light.

11. A method according to Claim 9 or Claim 10, wherein said specific frequency is approximately 8 KHz.

12. A method according to any one of Claims 9-11, wherein said detectable signal changes as said fluid sample is consumed.

13. A method according to any one of Claims 9-12, wherein said periodic determination is made at approximately 2 second intervals.

14. A method according to any one of Claims 9-13, wherein said periodic determination is made by observing light scatter at said inlet port.

15. A method for measuring an activity or determining an analyte in a fluid medium employing a device comprising at least one capillary unit acting as the motive force for moving the fluid medium in the device, at least one chamber unit, an inlet port, an outlet port distant from the inlet port, a reagent contained within the device, and a heater plate, said reagent being a member of a detection system, wherein said capillary acts as a metering pump and flow controller of the assay medium through said device to provide for a time controlled reaction with said reagent, said method comprising:

introducing a fluid sample though said inlet port into one of said units and allowing said fluid sample to transit from one unit to the next unit at a rate controlled by said capillary unit and react with said reagent resulting in a detectable signal produced by said detection system; and

determining said signal as a measure of said activity or the presence or concentration of said analyte in said sample,

wherein the temperature of said sample in said device is maintained at a substantially constant temperature by the method of repeating the following sequence steps;

measuring the ambient temperature;

measuring the temperature of said heater plate temperature;
updating a weighted average temperature;
updating a calculated cartridge temperature; and
adjusting the temperature of said heater plate to cause the calculated cartridge temperature to move closer to a desired cartridge temperature.

16. A method for measuring an activity or determining an analyte in a fluid medium employing a device comprising at least one capillary unit acting as the motive force for moving the fluid medium in the device, at least one chamber unit, an inlet port, an outlet port distant from the inlet port, and a reagent contained within the device, said reagent being a member of a detection system, wherein said capillary acts as a metering pump and flow controller of the assay medium through said device to provide for a time controlled reaction with said reagent, said method comprising:
introducing a fluid sample though said inlet port into one of said units and allowing said fluid sample to transit from one unit to the next unit at a rate controlled by said capillary unit and react with said reagent resulting in a detectable signal produced by said detection system;
determining said signal as a measure of said activity or the presence or concentration of said analyte in said sample;
reading an error detection coded signal from said device indicative of variations among a plurality of said devices which may affect said determination;
decoding said error detection coded signal; and
adjusting said determination in response to said error detection encoded signal.

17. A method as in Claim 16, wherein said error detection signal employs a Hamming code.

18. A method as in Claim 16, wherein said error detection coded signal is an optical signal.

19. A method as in Claim 18, which further comprises the step of measuring the level of ambient light to determine if said error detection encoded signal can be accurately read.

FIG. 1

EP 0 394 041 A2

FIG. 2

**FIG. 3**

START

WAIT FOR END OF CURRENT
20ms INTERNAL

V–S AMP=(SI63–V–S AMP)(0.0625)+V–S AMP    (SMOOTH)

FLOAT–TICK=FLOAT–TICK –I    (UPDATE–FLOAT–THRESHOLD)

FLOAT–TICK=0    N

Y

FLOAT–TICK=62

T–S AMP=(SAH–2–SEC –T–S AMP)
(0.0I56)+T–S AMP    ① (TAU–80 OR TAU–20)

FLOAT–THRESHOLD=T–S AMP/2    ② (UPDATE–FLOAT–
THRESHOLD) △

V–S AMP< FLOAT–THRESHOLD    N    (WAIT FOR MEASUREMENT)

Y

MEASUREMENT COMPLETE ③ □

HEATER CONTROL ALGORITHM

( DO THIS ONCE EVERY 2 SECONDS )

RTI=AMBIENT TEMPERATURE MEASUREMENT
RT2=HEATER PLATE TEMPERATURE MEASUREMENT
W-A-T = 46*RTI + I97*RT2 + I2 * 82
  (STANDS FOR WEIGHTED-AVERAGE-TEMPERATURE)
CCT=26*(WAT-CCT)+CCT
 (STANDS FOR CALCULATED CARTRIDGE TEMPERATURE)

IS CALCULATED CARTRIDGE TEMPERATURE
LESS THAN DESIRED OPERATING TEMPERATURE?    NO

YES

TURN ON HEATER FOR X SECONDS,
WHERE X IS LESS THAN 2

( DONE )

FIG. 4